(19) Europäisches Patentamt European Patent Office Office européen des brevets

(11) **EP 3 964 086 B1**

(12) **EUROPEAN PATENT SPECIFICATION**

(45) Date of publication and mention of the grant of the patent:
**15.05.2024 Bulletin 2024/20**

(21) Application number: **21193228.0**

(22) Date of filing: **26.08.2021**

(51) International Patent Classification (IPC):
**A24F 40/53** *(2020.01)* **A61M 11/04** *(2006.01)*
**A61M 15/06** *(2006.01)* **H05B 1/02** *(2006.01)*
**A24F 40/57** *(2020.01)*

(52) Cooperative Patent Classification (CPC):
(C-Sets available)
**A24F 40/53; A61M 11/042; A61M 15/0003;**
**A61M 15/0021; A61M 15/003; A61M 15/06;**
**H05B 1/0227;** A24F 40/57; A61M 2016/0021;
A61M 2016/0024; A61M 2202/0468;
A61M 2205/121; A61M 2205/123; A61M 2205/127;
A61M 2205/3317; (Cont.)

(54) **CONTROLLER FOR INHALATION DEVICE**

STEUERGERÄT FÜR INHALATIONSVORRICHTUNG

ORGANE DE COMMANDE POUR DISPOSITIF D'INHALATION

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **07.09.2020 JP 2020150106**

(43) Date of publication of application:
**09.03.2022 Bulletin 2022/10**

(73) Proprietor: **Japan Tobacco Inc.**
**Tokyo 105-6927 (JP)**

(72) Inventor: **ARADACHI, Takao**
**Tokyo, 130-8603 (JP)**

(74) Representative: **Hoffmann Eitle**
**Patent- und Rechtsanwälte PartmbB**
**Arabellastraße 30**
**81925 München (DE)**

(56) References cited:
**JP-A- 2020 058 337 US-A1- 2014 251 356**
**US-A1- 2020 237 010**

(52) Cooperative Patent Classification (CPC): (Cont.)
A61M 2205/3368; A61M 2205/3653;
A61M 2205/502; A61M 2205/8206

C-Sets
A61M 2202/0468, A61M 2202/0007

**Description**

BACKGROUND OF THE INVENTION

Field of the Invention

The present invention relates to a controller for an inhalation device.

Description of the Related Art

WO 2019/180909 A1 describes an aerosol generation device that generates an aerosol by heating an aerosol source using a heater and atomizing the aerosol source. In this device, based on the voltage or current of the heater measured by a sensor, it is estimated whether the remaining amount of the aerosol source is insufficient. If it is estimated that the remaining amount of the aerosol source is insufficient, power supply to the heater is stopped (more specifically, a switch connected to the heater is set in an OFF state). JP 2020-058337 A relates to a suction component generation device that can determine a low remaining amount state. The controller in this device determines the amount of power supplied to the load, the operating time of the load and/or the amount of suction component source consumed.
In the device that heats the aerosol source by the heater, it is required to further improve the safety.

SUMMARY OF THE INVENTION

The present invention provides, for example, a technique advantageous in improving the safety in energization control of a heater for heating an aerosol source.
The present invention is specified by independent claim 1. The dependent claims describe further preferred embodiments.
Further features of the present invention will become apparent from the following description of exemplary embodiments with reference to the attached drawings.

BRIEF DESCRIPTION OF THE DRAWINGS

Fig. 1 a view schematically showing an arrangement example of an inhalation device;
Fig. 2 is a view showing an arrangement example of electrical components of a controller;
Fig. 3 is a flowchart illustrating an operation example of the inhalation device;
Fig. 4 is a flowchart illustrating an example of a first detection-related process;
Fig. 5 is a flowchart illustrating an example of a second detection-related process; and
Fig. 6 is a graph for explaining the relationship among a first current value $TH_1$, a second current value $TH_2$, an upper limit value $k_{max}$ of the number of times k, and an upper limit value $n_{max}$ of the number of times n.

DESCRIPTION OF THE EMBODIMENTS

Hereinafter, embodiments will be described in detail with reference to the attached drawings. Note that the following embodiments are not intended to limit the scope of the claimed invention, and limitation is not made an invention that requires all combinations of features described in the embodiments. Two or more of the multiple features described in the embodiments may be combined as appropriate. Furthermore, the same reference numerals are given to the same or similar configurations, and redundant description thereof is omitted.

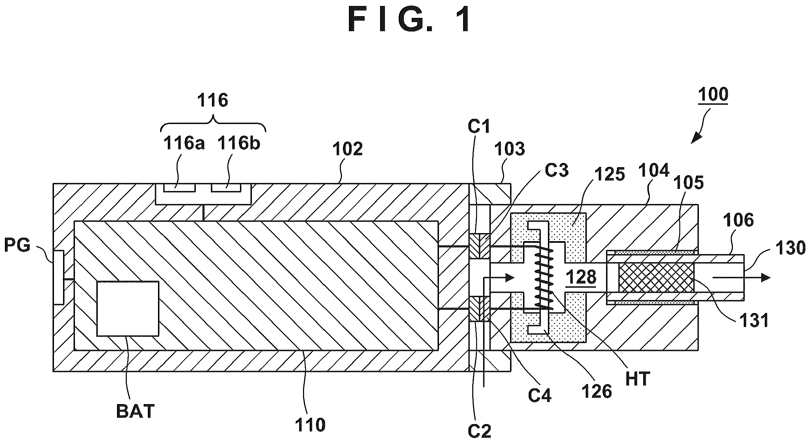

Fig. 1 schematically shows an arrangement example of a inhalation device (inhalator) 100 according to an embodiment. The inhalation device 100 can be configured to provide, to a user via a mouthpiece port 130, a gas containing an aerosol, a gas containing an aerosol and a flavor material, an aerosol, or an aerosol containing a flavor material in accordance with an operation requesting the aerosol (to be also referred to as an atomization request hereinafter) such as an inhalation operation by the user. The inhalation device 100 can comprise a controller 102 and an atomizer 104. The inhalation device 100 can comprise a holding portion 103 that detachably holds the atomizer 104. The controller 102 may be understood as a controller for the inhalation device. The atomizer 104 can be configured to atomize an aerosol source. The aerosol source can be, for example, a liquid such as a multivalent alcohol such as glycerin or propylene glycol. Alternatively, the aerosol source may contain a drug. The aerosol source can be a liquid, a solid, or a mixture of a liquid and a solid. A vapor source such as water may be used in place of the aerosol source.
The inhalation device 100 may further comprise a capsule 106 containing a flavor source 131. The atomizer 104 can include a capsule holder 105 that detachably holds the capsule 106. The capsule holder 105 may be included not in the atomizer 104 but in the controller 102. The flavor source 131 can be a molded body obtained by molding, for example, a cigarette material. Alternatively, the flavor source 131 may be made of a plant (for example, mint, herb, Chinese medicine, coffee beans, or the like) except the cigarette. A fragrance such as menthol may be added to the flavor source. The flavor source 131 may be added to an aerosol source. The atomizer 104 and the capsule holder 105 may be integrally formed in place of an arrangement in which the inhalation device 100 or the atomizer 104 includes the capsule holder 105.

The controller 102 can comprise electrical components 110 including a power supply BAT. The power supply BAT may be formed by a secondary battery such as a lithium ion secondary battery, or an electric double-layer capacitor such as a lithium ion capacitor. The electrical components 110 can include a user interface 116. Alternatively, the controller 102 may be understood to include the electrical components 110 and the user interface 116. The user interface 116 can include, for example, a display unit 116a (for example, a light emitting device such as an LED and/or an image display unit such as an LCD) that provides the user with information and/or an operation unit 116b (for example, a switch such as a button switch and/or a touch display) that accepts a user operation. The controller 102 can further comprise a connector PG electrically connected to the connector of an external power supply to enable charging by the external power supply. The connector PG is electrically connected to the electrical components 110. In the example shown in Fig. 1, the connector PG is provided at a position opposite to the position where the atomizer 104 is provided in the controller 102, but the position of the connector PG in the controller 102 can be arbitrary. The connector PG may be a female (concave type) receptacle or a male (convex type) plug.

The holding portion 103 of the controller 102 can include a first electrical contact C1 and a second electrical contact C2. In a state in which the atomizer 104 is held by the holding portion 103, the first electrical contact C1 of the holding portion 103 can contact a third electrical contact C3 of the atomizer 104, and the second electrical contact C2 of the holding portion 103 can contact a fourth electrical contact C4 of the atomizer 104. The controller 102 can supply power to the atomizer 104 (heater HT) via the first electrical contact C1 and the second electrical contact C2.

The atomizer 104 can include the third electrical contact C3 and the fourth electrical contact C4 described above. In addition, the atomizer 104 can include the heater HT for heating and atomizing the aerosol source, a container 125 for holding the aerosol source, and a transport portion (wick) 126 for transporting the aerosol source held by the container 125 to a heating region of the heater HT and holding the aerosol source in the heating region. At least part of the heating region can be arranged in a channel 128 formed in the atomizer 104. The first electrical contact C1, the third electrical contact C3, the heater HT, the fourth electrical contact C4, and the second electrical contact C2 form a current path for flowing the current to the heater HT. The transport portion 126 can be made of, for example, a fiber element such as a glass fiber, a porous material such as a ceramic, or a combination thereof. Note that the means for transporting the aerosol source held in the container 125 to the heating region is not limited to the wick, but a spraying device such as a spray or a transporting means such as a pump may be used instead.

As described above, the atomizer 104 can include the capsule holder 105 for detachably holding the capsule 106. As an example, the capsule holder 105 can hold the capsule 106 such that part of the capsule 106 is accommodated in the capsule holder 105 or the atomizer 104 and the remaining part of the capsule 106 including the mouthpiece port 130 is exposed. The user can hold the mouthpiece port 130 with his/her mouth and suck the gas containing the aerosol or the aerosol. Since the mouthpiece port 130 is included in the detachable capsule 106 as described above, the inhalation device 100 can be kept clean.

When the user holds the mouthpiece port 130 in the mouth and performs the inhalation operation, as exemplified by an arrow in Fig. 1, air flows into the channel 128 of the atomizer 104 through an opening (not shown). When the heater HT heats the aerosol source, the vaporized and/or aerosolized aerosol source is transported toward the mouthpiece port 130 with the air. In the process in which the aerosol source is transported toward the mouthpiece port 130, the vaporized and/or aerosolized aerosol source is cooled to form fine liquid droplets. This can promote aerosolization. In the arrangement in which the flavor source 131 is arranged, the flavor material generated by the flavor source 131 is added to this aerosol, and the resultant material is transported to the mouthpiece port 130, thus allowing the user to suck the aerosol containing the flavor material. Since the flavor material generated by the flavor source 131 is added to the aerosol, the flavor material can be efficiently transported to the lungs of the user without staying in the oral cavity.

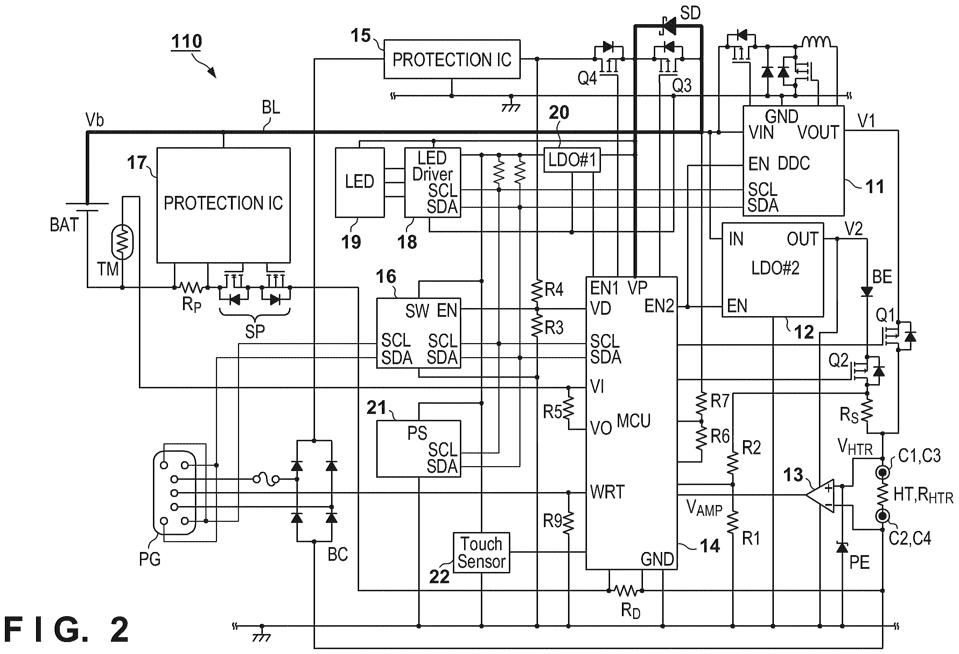

Fig. 2 shows an arrangement example of the electrical components 110 of the controller 102. Fig. 2 also shows the connector PG of the controller 102. The electrical components 110 can include, for example, the power supply BAT, a power supply unit that supplies power to the atomizer 104 (heater HT thereof), a detection unit for detecting the resistance value of the heater HT, and an energization control unit that controls energization of the heater HT in accordance with the information obtained by the detection unit. The heater HT has a resistance value $R_{HTR}$ that changes in accordance with the temperature of the heater HT. The resistance value may have a positive temperature coefficient characteristic (so-called PTC characteristic) and increase as the temperature of the heater HT increases, or may have a negative temperature coefficient characteristic (so-called NTC characteristic) and increase as the temperature of the heater HT decreases. As has been described above, the third electrical contact C3 of the heater HT contacts the first electrical contact C1 of the controller 102, and the fourth electrical contact C4 of the heater HT contacts the second electrical contact C2 of the controller 102.

The power supply unit that supplies power to the heater HT can include a voltage converter 11 and a switch Q1 on the power supply line from the power supply BAT to the heater HT. The voltage converter 11 (first regulator) includes, for example, a DC/DC converter. The voltage converter 11 converts a voltage Vb supplied from the plus terminal of the power supply BAT into a heater driving voltage V1, and outputs it from an output terminal VOUT. The heater driving voltage V1 output from the output terminal VOUT of the voltage converter 11 is supplied to the first electrical contact C1 contacting the third electrical contact C3 of the heater HT. Since the second electrical contact C2 contacting the fourth electrical contact C4 of the heater HT is electrically connected to the minus terminal of the power supply BAT, the current path for flowing the current to the heater HT can be formed between the output terminal VOUT of the voltage converter 11 and the minus terminal of the power supply BAT. The generated amount of aerosol tends to increase as the voltage applied to the heater HT is higher. Therefore, the voltage converter 11 preferably includes a boost DC/DC converter or a buck-boost DC/DC converter. The switch Q1 includes, for example, a field effect transistor (FET), and opening/closing (ON/OFF) of the switch Q1 can be controlled by a processor 14. The switch Q1 can be arranged on the line (current path) connecting the output terminal VOUT of the voltage converter 11 and the heater HT (first electrical contact C1), but the present invention is not limited to this, and the switch Q1 may be arranged on the line connecting the heater HT (second electrical contact C2) and the minus terminal of the power supply BAT. Note that the diode added to the switch Q1 in Fig. 2 represents the body (parasitic) diode of the field effect transistor.

The detection unit for detecting the resistance value $R_{HTR}$ of the heater HT can include a voltage conversion circuit 12 and an amplifier 13. The voltage conversion circuit 12 (second regulator) includes, for example, a linear regulator such as an LDO (Low DropOut) regulator. The voltage conversion circuit 12 converts the voltage Vb supplied from the plus terminal of the power supply BAT into a detection voltage V2 for detecting the resistance value $R_{HTR}$ of the heater HT, and outputs it from an output terminal VOUT. The amplifier 13 can include, for example, an operational amplifier which includes a noninverting input terminal, an inverting input terminal, and an output terminal. The positive power supply terminal of the amplifier 13 can be connected to the output terminal VOUT of the voltage conversion circuit 12, and the negative power supply terminal thereof can be connected to the ground line. The noninverting input terminal of the amplifier 13 is connected to the first electrical contact C1, and the inverting input terminal thereof is connected to the second electrical contact C2. Accordingly, the amplifier 13 can amplify the potential difference between the first electrical contact C1 and the second electrical contact C2, that is, a voltage $V_{HTR}$ generated in the heater HT, and output it as an output voltage $V_{AMP}$. The output voltage $V_{AMP}$ of the amplifier 13 can be input to the processor 14. Note that in the example shown in Fig. 2, a Zener diode PE is provided between the noninverting input terminal of the amplifier 13 and the ground line. The Zener diode PE is used to suppress an unexpected operation or failure of the amplifier 13 caused by an input of an excessive voltage to the noninverting input terminal of the amplifier 13.

The detection unit for detecting the resistance value $R_{HTR}$ of the heater HT can further include a switch Q2 and a shunt resistor Rs ("Rs" may also refer to the resistance value of the shunt resistor Rs hereinafter). Assume that the resistance value of the shunt resistor Rs hardly changes even when the temperature of the shunt resistor Rs changes. The switch Q2 includes, for example, a field effect transistor (FET), and opening/closing (ON/OFF) of the switch Q2 can be controlled by the processor 14. The switch Q2 can be arranged on the line connecting the output terminal OUT of the voltage conversion circuit 12 and the heater HT (first electrical contact C1), but the present invention is not limited to this, and the switch Q2 may be arranged on the line connecting the heater HT (second electrical contact C2) and the minus terminal of the power supply BAT. A diode BE can be provided on the line connecting the output terminal OUT of the voltage conversion circuit 12 and the switch Q2. The shunt resistor Rs can be arranged on the line connecting the switch Q2 and the heater HT in series with the switch Q2. Note that the diode added to the switch Q2 in Fig. 2 represents the body (parasitic) diode of the field effect transistor. In the example shown in Fig. 2, resistors R1 and R2 arranged in series are provided between the line connecting the switch Q2 and the shunt resistor Rs and the ground line, and the voltage between the resistor R1 and the resistor R2 is supplied to the processor 14.

The noninverting input terminal of the amplifier 13 is connected between the shunt resistor Rs and the heater HT, and the series circuit of the shunt resistor Rs and the heater HT is connected between the output terminal OUT of the voltage conversion circuit 12 and the minus terminal of the power supply BAT. That is, a voltage obtained by dividing the detection voltage V2 (the voltage obtained by subtracting a forward voltage Vf of the diode BE to be described later therefrom) by the shunt resistor Rs and the heater HT is input to the noninverting input terminal of the amplifier 13. Since the resistance value $R_{HTR}$ changes in accordance with the temperature of the heater HT, according to the arrangement example of the electrical components 110 of the controller 102 shown in Fig. 2, the amplifier 13 can output the output voltage $V_{AMP}$ that changes in accordance with the temperature of the heater HT.

In order to detect the resistance value $R_{HTR}$ of the heater HT, the switch Q1 is turned off and the switch Q2 is turned on. In this embodiment, after the switch Q1 is turned on to supply power to the heater HT in accordance with an atomization request from the user, the switch Q2 is turned on and then the switch Q1 is turned off. At this time, letting Vf be the forward voltage of the diode BE and $I_{HTR}$ be the current flowing through the heater HT, the resistance value

$R_{HTR}$ of the heater HT is expressed by equation (1):

$$R_{HTR} = V_{HTR}/I_{HTR} = V_{HTR}\cdot(R_{HTR} + R_S)/(V2 - Vf) \qquad ...(1)$$

By modifying equation (1), equation (2) giving the resistance value $R_{HTR}$ is obtained:

$$R_{HTR} = R_S\cdot V_{HTR}/(V2 - Vf - V_{HTR}) \qquad ...(2)$$

If the amplifier 13 of the detection unit has an amplification factor A, the output voltage $V_{AMP}$ of the amplifier 13 is given by equation (3):

$$V_{AMP} = A\cdot V_{HTR} \qquad ...(3)$$

By modifying equation (3), equation (4) giving the voltage $V_{HTR}$ of the heater HT is obtained:

$$V_{HTR} = V_{AMP}/A \qquad ...(4)$$

Thus, the resistance value $R_{HTR}$ of the heater HT can be obtained according to equation (2) and equation (4). Note that the switch Q2 is turned off after the output voltage $V_{AMP}$ of the amplifier 13 used to detect the resistance value $R_{HTR}$ of the heater HT is obtained.

The energization control unit that performs energization control of the heater HT can include the processor 14. The processor 14 can be operated by the voltage Vb of the power supply BAT. More specifically, as indicated by a bold line BL in Fig. 2, the voltage Vb of the power supply BAT can be applied to a power supply terminal VP of the processor 14 via the line connecting the plus terminal of the power supply BAT and the power supply terminal VP of the processor 14. A diode SD arranged in parallel with a switch Q3 can be provided on the bold line BL. The diode SD is, for example, a Schottky barrier diode, and can be arranged in parallel with the switch Q3. Since the forward voltage of the Schottky barrier diode tends to be smaller than the forward voltage of the body diode, the Schottky barrier diode enables highly efficient power supply from the power supply BAT to the power supply terminal VP of the processor 14. Here, the processor 14 can be formed by, for example, an MCU (Micro Controller Unit), but may be formed by an MCU and an analog circuit.

The processor 14 generates a control signal for controlling energization of the heater HT in accordance with the information obtained by the above-described detection unit (here, the output voltage $V_{AMP}$ of the amplifier 13). The control signal can be, for example, a signal for controlling opening/closing of the switch Q1, but can include another control signal (for example, a control signal for controlling the display unit 116a). The control signal may be, for example, a control signal for suppressing overheating of the heater HT, or may be a control signal for converging the temperature of the heater HT to a target temperature. Based on the voltage generated in a resistor $R_D$ (first resistor) arranged on the line connecting the minus terminal of the power supply BAT and the heater HT (second electrical contact C2), the processor 14 can detect a current value $I_D$ flowing through the resistor $R_D$, that is, the current value $I_{HTR}$ of the heater HT. If an overcurrent is detected in the heater HT, the processor 14 can perform a process of stopping the energization of the heater HT by turning off the switch Q1, or the like.

Based on the resistance value $R_s$, the voltage Vf, the voltage V2, and the output voltage $V_{AMP}$ of the amplifier 13, the processor 14 can calculate the resistance value $R_{HTR}$ of the heater HT according to the above-described equation (2) and equation (4). The resistance value $R_s$, the voltage Vf, and the voltage V2 are known values. Then, the processor 14 calculates an estimated temperature $T_{HTR}$ of the heater HT according to following equation (5). The processor 14 can control opening/closing of the switch Q1 based on the calculated estimated temperature $T_{HTR}$ so that the temperature of the heater HT matches or converges to the target temperature.

$$T_{HTR} = T_{ref} + (1/\alpha)\cdot(R_{HTR} - R_{ref})\cdot(1/R_{ref})\cdot10^6 \qquad ...(5)$$

In equation (5), $T_{ref}$ is the reference temperature of the heater HT. $R_{ref}$ is the reference resistance value of the heater HT, and this is the resistance value $R_{HTR}$ of the heater HT at the reference temperature. $\alpha$ is the temperature coefficient [ppm/°C] of the heater HT, and this is a known value. Here, the reference temperature can be an arbitrary temperature, and can be stored in a memory of the processor 14 in association (linking) with the reference resistance value. As the reference temperature, the preset temperature may be used, or the temperature of the heater HT obtained upon acquiring the reference resistance value may be used. The temperature of the heater HT obtained upon acquiring the reference resistance value may be obtained by applying the estimated temperature $T_{HTR}$ of the heater HT newly calculated using the above-described equations (1) to (5), or may be obtained by converting the output of the sensor (for example, a temperature sensor TM) that detects the temperature of an arbitrary portion in the inhalation device 100.

The electrical components 110 of the controller 102 can further comprise a charging circuit for controlling charging of the power supply BAT by an external power supply when the connector PF of the controller 102 is connected to the connector of the external power supply. The charging circuit can include, for example, a bridge circuit BC, a protection circuit 15, the switch Q3, and a switch Q4. The bridge circuit BC is a circuit that allows the controller 102 to operate normally even if the connector PG is connected to the connector of the external power supply while being inverted (for example, the vertical direction in the drawing is inverted). The bridge circuit BC can be formed by, for example, four diodes. The protection circuit 15 is a circuit for preventing an overcurrent from flowing to the power supply BAT of the controller 102 from the external power supply via the connector PG.

The switches Q3 and Q4 are arranged in series on the line connecting the bridge circuit BC and the plus terminal of the power supply BAT. Each of the switches Q3 and Q4 includes, for example, a field effect transistor (FET), and opening/closing (ON/OFF) of the switches Q3 and Q4 can be controlled by the processor 14. When the connector PG is connected to the connector of the external power supply, the processor 14 turns on the switch Q3, and this enables charging of the power supply BAT from the external power supply via the connector PG. Note that the diode added to each of the switches Q3 and Q4 in Fig. 2 represents the body (parasitic) diode of the field effect transistor. The processor 14 may perform dropper control in which, by controlling ON/OFF of the switch Q3, power unnecessary for charging the power supply BAT is discarded as heat from the power supplied from the connector PG. When the processor 14 performs the dropper control using the switch Q3, it is possible to highly control the charging of the power supply BAT without using a dedicated charging IC or the like.

The electrical components 110 of the controller 102 can further comprise a switch circuit 16 and a protection circuit 17. The switch circuit 16 is a circuit that enables communication between the processor 14 and the external power supply when the connector PG is connected to the connector of the external power supply and a predetermined voltage is applied to the EN terminal. The protection circuit 17 detects, based on the voltage generated in a resistor $R_p$ (second resistor) arranged on the power supply line to the heater HT (in the arrangement example shown in Fig. 2, the line connecting the minus terminal of the power supply BAT and the heater HT (second electrical contact C2)), the current flowing through the resistor $R_p$, that is, the current of the heater HT. If an overcurrent is detected in the heater HT, the protection circuit 17 performs a process of stopping the energization of the heater HT, or the like. For example, a switch circuit SP formed by field effect transistors or the like is provided on the power supply line to the heater (in the arrangement example shown in Fig. 2, the line connecting the minus terminal of the power supply BAT and the heater HT (second electrical contact C2)). If an overcurrent is detected, the protection circuit 17 can stop the energization of the heater HT by turning off the switch circuit SP. Note that the protection circuit 17 can be configured to operate independently of control of the processor 14.

The electrical components 110 of the controller 102 can further comprise an LED driving circuit 18, a voltage conversion circuit 20, a puff sensor 21, a touch sensor 22, and the temperature sensor TM. The LED driving circuit 18 drives an LED 19 that forms the display unit 116a of the user interface 116. The voltage conversion circuit 20 includes, for example, a linear regulator such as an LDO (Low DropOut) regulator. The voltage conversion circuit 20 converts the voltage Vb supplied from the plus terminal of the power supply BAT into a voltage to be input to the switch 16 and the puff sensor 21, and outputs it. The puff sensor 21 (for example, a pressure sensor or a microphone condenser) detects a puff operation of the user, and supplies the detection signal to the processor 14. The detection of the puff operation using the puff sensor 21 is a specific example of the atomization request described above. The touch sensor 22 forms the operation unit 116b of the user interface 116. If an operation (for example, a touch operation) by the user is detected, the touch sensor 22 supplies the detection signal to the processor 14. The touch operation on the touch sensor 22 is a specific example of the atomization request described above. The temperature sensor TM is provided to detect the temperature of the power supply BAT, and can include, for example, a thermistor whose resistance value changes in accordance with the temperature. The processor 14 measures the voltage divided by a resistor R5 connected in series with the thermistor serving as the temperature sensor TM and the thermistor to obtain the resistance value of the thermistor. Based on the resistance value of the thermistor, the processor 14 can calculate the temperature of the power supply BAT. Preferably, the temperature sensor TM is installed near the power supply BAT or on the surface of the power supply BAT.

Here, in the example shown in Fig. 2, the line connecting the bridge circuit BC (protection circuit 15) and the switch Q4 and the ground line are connected via the resistors R3 and R4. The voltage between the resistor R3 and the resistor R4 can be input to the EN terminal of the switch circuit 16 and a voltage detection terminal VD of the processor 14. The voltage detection terminal VD of the processor 14 is a terminal for detecting whether the voltage of the external power supply is applied (that is, whether power is supplied from the external power supply). If a voltage equal to or higher than a predetermined threshold value is detected at the voltage detection terminal VD, the processor 14 can determine that the voltage is applied from the external power supply. The voltage Vb supplied from the plus terminal of the power supply BAT can be input to the processor 14 via resistors R6 and R7. The voltage between the resistor R6 and the resistor R7 can also be input to the processor 14.

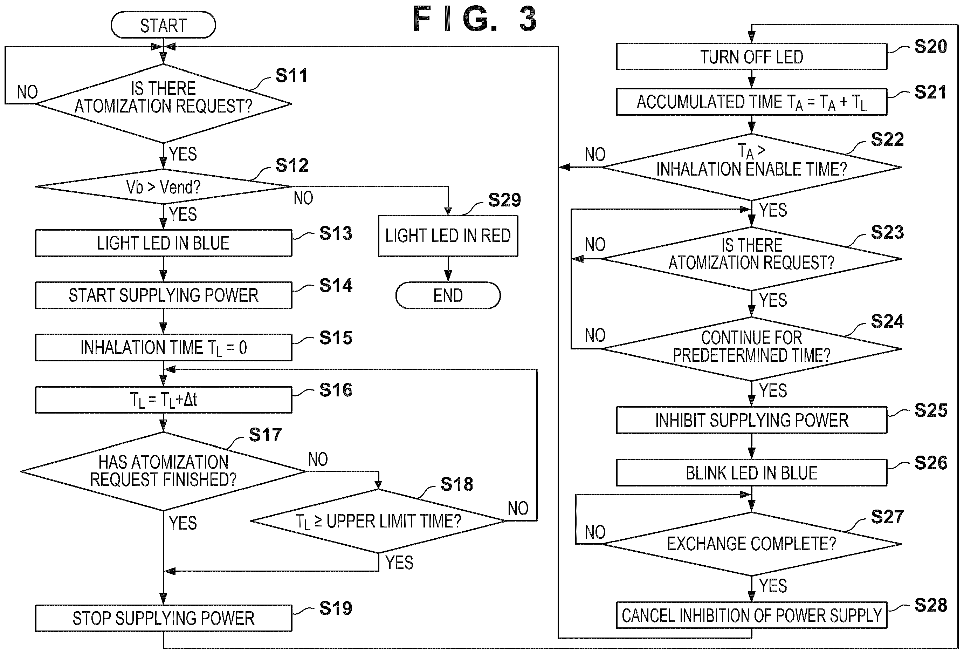

Fig. 3 illustrates an operation example of the inhalation device 100. This operation is a process (atomization process) of heating the aerosol source by the heater HT in accordance with an atomization request from the user and providing the atomized aerosol source from the mouthpiece port 130, and controlled by the processor 14. The processor 14 includes a memory storing programs, and a CPU that operates in accordance with the programs.

In step S11, the processor 14 waits for reception of an atomization request (more specifically, a detection signal transmitted from the puff sensor 21 and/or the touch sensor 22). If the atomization request is received, the processor 14 executes step S12. The atomization request is a request to operate the atomizer 104, more specifically, a request to control the heater HT within a target temperature range so as to generate an aerosol from the aerosol source. The atomization request can be an operation of detecting by the puff sensor 21 that the user has performed an inhalation operation (puff operation) through the mouthpiece port 130, and notifying the processor 14 of the detection by the puff sensor 21 (for example, transmission of a detection signal). Alternatively, the atomization request can be an operation of notifying, by the operation unit 116b, the processor 14 that the user has operated the operation unit 116b (touch sensor 22) (for example, transmission of an operation signal). Hereinafter, during the inhalation operation by the user or during the operation of the operation unit 116b by the user, the atomization request is continuously transmitted from the puff sensor 21 or the operation unit 116b, and the atomization request (transmission thereof) ends when the user terminates the inhalation operation or the operation of the operation unit 116b.

In step S12, the processor 14 obtains the voltage Vb of the power supply BAT from a power supply management circuit (not shown), and determines whether the voltage Vb exceeds a discharge end voltage Vend (for example, 3.2 V). If the voltage Vb is equal to or lower than the discharge end voltage Vend, this means that the dischargeable remaining amount of the power supply BAT is insufficient. Accordingly, if the voltage Vb is equal to or lower than the discharge end voltage Vend, the process advances to step S29, and the processor 14 gives a notification to prompt charging of the power supply BAT by using the display unit 116a (LED 19) of the user interface 116. For example, this notification can be lighting in red or blinking the LED 19 included in the display unit 116a. When the notification is given, the user connects the controller 102 (connector PG) to the external power supply. With this, the power supply BAT of the controller 102 is charged, and the dischargeable remaining amount can be increased. On the other hand, if the voltage Vb exceeds the discharge end voltage Vend in step S12, the processor 14 performs a heating process. The heating process is a process of controlling the switch Q1 to supply power to the heater HT in accordance with the reception of the atomization request of the aerosol source, thereby heating the aerosol source. The heating process can include steps S13 to S17.

In step S13, the processor 14 can notify, using the display unit 116a (LED 19) of the user interface 116, that a normal operation is possible. For example, this notification can be lighting, in blue, of the LED 19 included in the display unit 116a. Then, in step S14, the processor 14 starts power supply control of the heater HT. The power supply control of the heater HT includes temperature control of controlling the heater HT within the target temperature range. The temperature control can include feedback control of calculating the estimated temperature $T_{HTR}$ of the heater HT by detecting the resistance value $R_{HTR}$ of the heater HT, and controlling opening/closing of the switch Q1 based on the estimated temperature $T_{HTR}$ such that the temperature of the heater HT falls within the target temperature range (for example, the temperature of the heater HT matches or converges to the target temperature).

Then, in step S15, the processor 14 resets an inhalation time $T_L$ to 0. After that, in step S16, the processor 14 adds $\Delta t$ to the inhalation time $T_L$. $\Delta t$ corresponds to the time interval between the execution of step S16 and the next execution of step S16.

Then, in step S17, the processor 14 determines whether the atomization request has finished. If the atomization request has finished, the processor 14 advances to step S19, and stops the power supply control of the heater HT. On the other hand, if the atomization request has not finished, the processor 14 advances to step S18, and determines whether the inhalation time $T_L$ has reached the upper limit time. If the inhalation time $T_L$ has not reached the upper limit time, the processor 14 returns to step S16. If the inhalation time $T_L$ has reached the upper limit time, the processor 14 advances to step S19. As an example, the upper limit time may be between 2.0 and 2.5 sec.

After step S19, in step S20, the processor 14 turns off the LED 19 which has been lit in blue. The order of step S19 and step S20 may be reversed, or the processor 14 may simultaneously execute steps S19 and S20. Then, in step S21, the processor 14 updates an accumulated time $T_A$. More specifically, the inhalation time $T_L$ is added to the current accumulated time $T_A$ in step S21. The accumulated time $T_A$ can be the accumulated time of the capsule 106 used for inhalation. In other words, the accumulated time $T_A$ can be the accumulated time of inhalation of the aerosol via the flavor source 131 of the capsule 106.

In step S22, the processor 14 determines whether the accumulated time $T_A$ does not exceed the inhalation enable time (for example, 120 sec). If the accumulated time $T_A$ does not exceed the inhalation enable time, this means that the capsule 106 can still provide the flavor material, so that the process returns to step S11. On the other hand, if the accumulated time $T_A$ exceeds the inhalation enable time, the process advances to step S23, and the processor 14 waits for generation of an atomization request. If an atomization request is generated, in step S24, the processor 14 waits for the atomization request to continue for a predetermined time. Thereafter, in step S25, the processor 14 inhibits the power supply control of the heater HT. Note that step S24 may be omitted.

Then, in step S26, the processor 14 gives a notification to prompt an exchange of the capsule 106 by using the display unit 116a of the user interface 116. For example, this notification can be blinking (repetition of turning on and off), in blue, of the LED 19 included in the display unit 116a. When the notification is given, the user can exchange the capsule 106. In an example, one atomizer 104 and a plurality of (for example, three) capsules 106 can be sold as one set. In such an example, after the one atomizer 104 and all the capsules 106 included in one set are consumed, the atomizer 104 and the last capsule 106 included in the consumed set can be exchanged with the atomizer 104 and the capsule 106 included in a new set. The order of step S25 and step S26 may be reversed, or the processor 14 may simultaneously execute steps S25 and S26.

In step S27, the processor 14 waits for completion of the exchange of the capsule 106 (or the capsule 106 and the atomizer 104). After the exchange of the capsule 106 is completed, the process advances to step S28, and the processor 14 cancels the inhibition of the power supply control of the heater HT and returns to step S11.

In the controller 102 described above, by setting a first constraint condition, a second constraint condition, and a third constraint condition for the energization control of the heater HT, the safety of the energization control is improved. If at least one of the first constraint condition, the second constraint condition, and the third constraint condition is met, the processor 14 restricts (for example, stops) energization to the heater HT even if the atomization request is detected by the puff sensor 21 and/or the touch sensor 22. In this manner, by setting three or more constraint conditions for the energization control of the heater HT, the safety of the inhalation device 100 (controller 102) can be improved.

Here, monitoring targets for determining whether to restrict energization to the heater HT are preferably different among the first constraint condition, the second constraint condition, and the third constraint condition. In this manner, by individually setting the constraint conditions for three or more monitoring targets different from each other, it is possible to detect an abnormality of the controller 102 (inhalation device 100) from the three or more viewpoints different from each other. Therefore, the safety of the controller 102 (inhalation device 100) can be more improved. For example, the first constraint condition can be a constraint condition regarding the inhalation time $T_L$. In this case, the monitoring target of the first constraint condition is the atomization request from the user detected by the puff sensor 21 and/or the touch sensor 22 (more specifically, the detection signal transmitted from the puff sensor 21 and/or the touch sensor 22). The second constraint condition can be a constraint condition regarding the current flowing through the heater HT. In this case, the monitoring target of the second constraint condition is the current value $I_D$ of the resistor $R_D$ (that is, the current $I_{HTR}$ of the heater HT) detected based on the voltage of the resistor $R_D$. The third constraint condition can be a constraint condition regarding the temperature of the heater HT. In this case, the monitoring target of the third constraint condition is the temperature (estimated temperature $T_{HTR}$) of the heater HT calculated based on the output voltage $V_{AMP}$ of the amplifier 13 (that is, the resistance value $R_{HTR}$ of the heater HT).

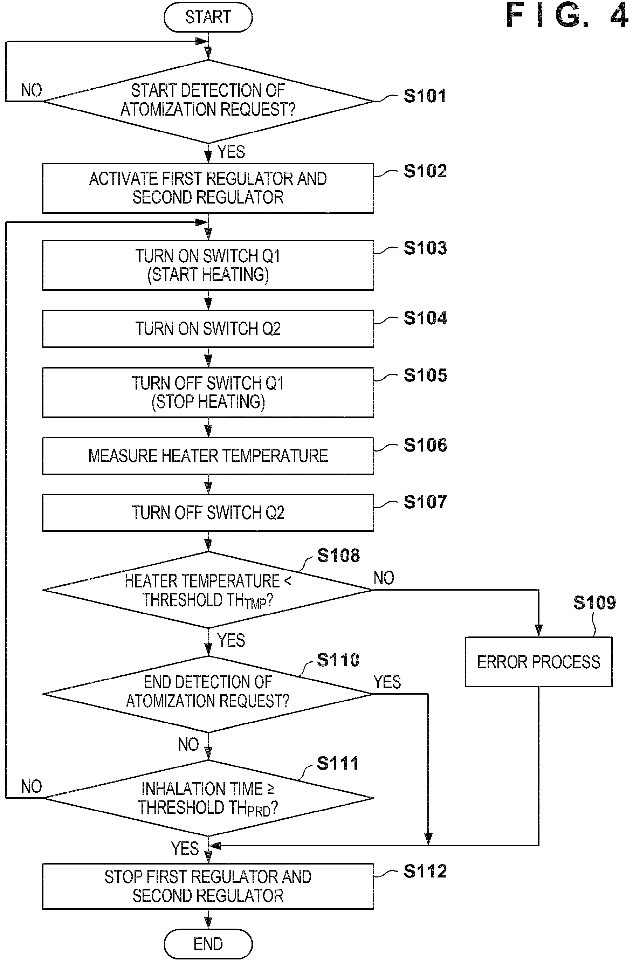

The detection-related process related to detection of an abnormality of the controller 102 will be described below. Fig. 4 illustrates a first detection-related process for detecting an abnormality regarding the inhalation time $T_L$ and the temperature of the heater HT. The first detection-related process illustrated in Fig. 4 can be performed by the processor 14 in parallel with the process (atomization process) illustrated in Fig. 3. More specifically, the first detection-related process illustrated in Fig. 4 can be performed between step S14 and step S19 of the process illustrated in Fig. 3. Alternatively, the process from step S102 to step S112 of the first detection-related process illustrated in Fig. 4 may be the specific mode of the process from step S14 to step S19 of the process illustrated in Fig. 3.

In step S101, the processor 14 determines whether detection of an atomization request is started. If detection of an atomization request is started, the process advances to step S102. For example, based on whether reception of a detection signal from the puff sensor 21 and/or the touch sensor 22 is started, the processor 14 can determine whether detection of an atomization request is started. Then, in step S102, the processor 14 activates the voltage converter 11 (first regulator) and the voltage conversion circuit 12 (second regulator) by setting the EN terminal of each of the voltage converter 11 and the voltage conversion circuit 12 to High level.

In step S103, the processor 14 turns on the switch Q1 to apply the voltage V1 output from the output terminal VOUT of the voltage converter 11 to the heater HT, thereby starting heating of the aerosol source by the heater HT. Further, the processor 14 turns on the switch Q2 in step S104 and then turns off the switch Q1 in step S105, thereby stopping the heating of the aerosol source by the heater HT. The order of step S104 and step S105 may be reversed, or the processor 14 may simultaneously execute step S104 and step S105. In this state, detection of the resistance value $R_{HTR}$ of the heater HT is enabled. In step S106, the processor 14 measures the temperature (estimated temperature $T_{HTR}$) of the heater HT based on the resistance value $R_{HTR}$ of the heater HT. More specifically, based on the output voltage $V_{AMP}$ of the amplifier 13, the processor 14 calculates the resistance value $R_{HTR}$ of the heater HT according to equation (2) and equation (4), and based on the calculated resistance value $R_{HTR}$, calculates the temperature (estimated temperature $T_{HTR}$) of the heater HT according to equation (5). In step S107, the processor 14 turns off the switch Q2.

The order of steps S106 and step S107 may be reversed, or the processor 14 may simultaneously execute step S106 and step S107.

In step S108, the processor 14 determines whether the temperature (estimated temperature $T_{HTR}$) of the heater HT measured in step S106 is lower than a threshold value $TH_{TMP}$. If the temperature of the heater HT is equal to or higher than the threshold value $TH_{TMP}$, the processor 14 performs an error process in step S109 and then advances to step S112. In step S112, the processor 14 sets the EN terminal of each of the voltage converter 11 (first regulator) and the voltage conversion circuit 12 (second regulator) to Low level, thereby stopping the voltage converter 11 and the voltage conversion circuit 12. In this manner, step S108 corresponds to the constraint condition (third constraint condition) for restricting the energization of the heater HT. If the constraint condition that the temperature of the heater HT is equal to or higher than the threshold value $TH_{TMP}$ is met, the processor 14 stops the energization to the heather HT even if the atomization request is detected by the puff sensor 21 and/or the touch sensor 22.

Here, as the error process in step S109, the processor 14 gives a notification indicating that an error (abnormality) has occurred in the controller 102, for example, by using the display unit 116a of the user interface 116. For example, this notification can be lighting or blinking of the LED 19 by the LED driving circuit 18. The threshold value $TH_{TMP}$ is the upper limit value of the temperature and can be arbitrarily set. As an example, the threshold value $TH_{TMP}$ can be set to 250°C, 300°C, or an arbitrary temperature between 250°C and 300°C.

In step S108, if the temperature of the heater HT is lower than the threshold value $TH_{TMP}$ (that is, if the constraint condition that the temperature of the heater HT is equal to or higher than the threshold value $TH_{TMP}$ is not met), the process advances to step S110. In step S110, the processor 14 determines whether the detection of the atomization request has finished (for example, whether reception of the detection signal from the puff sensor 21 and/or the touch sensor 22 has finished). If the detection of the atomization request has finished, the process advances to step S112, and the processor 14 sets the EN terminal of each of the voltage converter 11 and the voltage conversion circuit 12 in Low level, thereby stopping the voltage converter 11 and the voltage conversion circuit 12. On the other hand, if the detection of the atomization request has not finished, the process advances to step S111.

In step S111, the processor 14 determines whether the inhalation time $T_L$ is equal to or longer than a threshold value $TH_{PRD}$. The inhalation time $T_L$ may be understood as the request continuation time (detection time) during which the atomization request is continuously detected by the puff sensor 21 and/or the touch sensor 22. If the inhalation time $T_L$ is equal to or longer than the threshold value $TH_{PRD}$, the process advances to step S112, and the processor 14 sets the EN terminal of each of the voltage converter 11 and the voltage conversion circuit 12 to Low level, thereby stopping the voltage converter 11 and the voltage conversion circuit 12. That is, step S111 corresponds to the constraint condition (first constraint condition) for restricting the energization of the heater HT. If the constraint condition that the inhalation time $T_L$ is equal to or longer than the threshold value $TH_{PRD}$ (first threshold value) is met, the processor 14 stops the energization to the heather HT even if the atomization request is detected by the puff sensor 21 and/or the touch sensor 22. On the other hand, if the inhalation time $T_L$ is shorter than the threshold value $TH_{PRD}$, the process returns to step S103. Here, the threshold value $TH_{PRD}$ is the upper limit value of the time and can be arbitrarily set. As an example, the threshold value $TH_{PRD}$ may be between 2.0 and 2.5 sec like the upper limit time used in step S17 of Fig. 3.

Here, if the temperature of the heater HT is equal to or higher than the threshold value $TH_{TMP}$ in step S108 or if the inhalation time $T_L$ is equal to or longer than the threshold value $TH_{PRD}$ in step S111, the processor 14 may perform an energization inhibition process of inhibiting the energization to the heater HT in step S112. The energization inhibition process can include, for example, a process of inhibiting the voltage converter 11 from outputting the heater driving voltage V1 and/or a process of inhibiting turn-on of the switch Q1 (that is, connecting the power supply line of the heater HT by the switch Q1). The energization inhibition process may be performed for a predetermined period (that is, the inhibition of the energization to the heater HT may be maintained for the predetermined period).

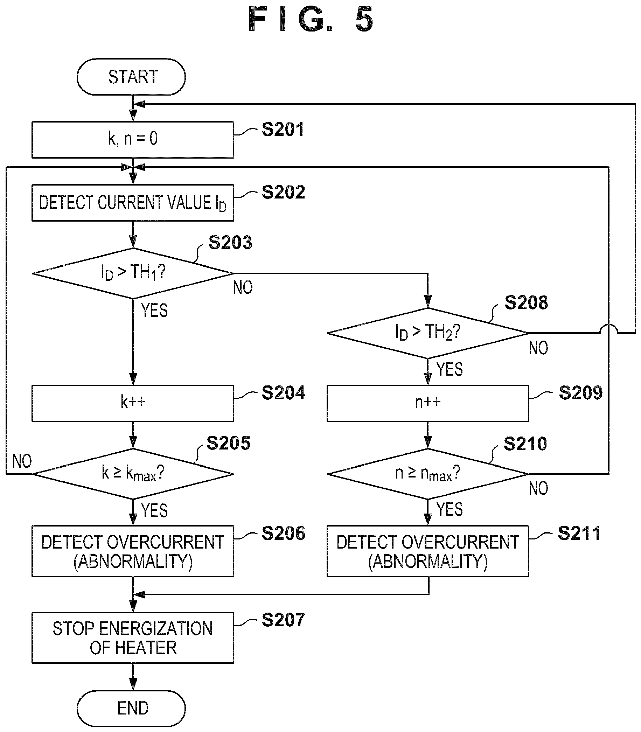

Fig. 5 illustrates a second detection-related process for detecting an abnormality regarding the current of the heater HT. The second detection-related process illustrated in Fig. 5 can be performed by the processor 14 in parallel with the process (atomization process) illustrated in Fig. 3 and the first detection-related process illustrated in Fig. 4.

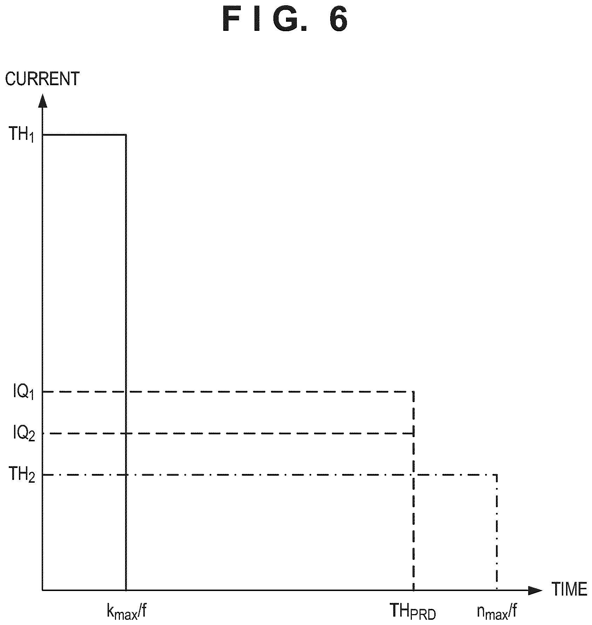

In the second detection-related process, an overcurrent of the heater HT can be detected based on the number of times k the current value $I_D$ of the resistor $R_D$ continuously exceeds a first current value $TH_1$ and the number of times n the current value $I_D$ continuously exceeds a second current value $TH_2$. Note that in this embodiment, the processor 14 obtains the current value $I_D$ such that the current (discharge current) flowing from the heater HT (second electrical contact C2) toward the negative electrode of the power supply BAT has a positive value. Note that the processor 14 may obtain the current value $I_D$ such that, in addition to the discharge current, the current (charging current) flowing in the opposite direction to the discharge current also has a positive value. Fig. 6 shows the relationship among the first current value $TH_1$, the second current value $TH_1$, an upper limit value $k_{max}$ of the number of times k, and an upper limit value $n_{max}$ of the number of times n. The ordinate in Fig. 6 represents the current value, and the abscissa represents time. By dividing the upper limit value $k_{max}$ of the number of times k and the upper limit value $n_{max}$ of the number of times n by a sampling frequency f (repetition frequency), they can be converted into time (first time $k_{max}/f$ and second time $n_{max}/f$). Fig. 6 also shows the threshold value $TH_{PRD}$ of the inhalation time $T_L$ (that is, the detection time of the atomization request).

The first current value $TH_1$ is a current threshold value set to detect a temporary (instantaneous) overcurrent of the heater HT. Therefore, the first current value $TH_1$ can be set to a value larger than the current value (to be sometimes referred to as the normal current value hereinafter) flowing through the heater HT during normal energization of the heater HT. For example, a normal current value $I_{Q1}$ of the heater HT via the switch Q1 can be given by following equation (6), and a normal current value $I_{Q2}$ of the heater HT via the switch Q2 can be given by following equation (7). The first current value $TH_1$ can be set to a value larger than the total value of the normal current value $I_{Q1}$ and the normal current value $I_{Q2}$. In addition, since the first current value $TH_1$ is used to detect a temporary overcurrent, the upper limit value $k_{max}$ of the number of times k can be set such that the first time $k_{max}/f$ is shorter than the threshold value $TH_{PRD}$ of the inhalation time $T_L$.

$$I_{Q1} = V1/R_{HTR} \qquad\qquad ...(6)$$

$$I_{Q2} = (V2 - Vf)/(R_s + R_{HTR}) \qquad\qquad ...(7)$$

The second current value $TH_2$ is set to detect a long-term overcurrent of the heater HT that can be generated due to an abnormality of the switch Q1 or Q2, or the like. More specifically, the second current value $TH_2$ can be set to detect an overcurrent of the heater HT after the inhalation time $T_L$ reaches the threshold value $TH_{PRD}$ and the energization of the heater HT is stopped. Therefore, the second current value $TH_2$ can be set to a value smaller than the normal current values (each of the normal current value $I_{Q1}$ and the normal current value $I_{Q2}$). The upper limit value $n_{max}$ of the number of times n can be set such that the second time $n_{max}/f$ (second threshold value) is longer than the threshold value $TH_{PRD}$ (first threshold value) of the inhalation time $T_L$. With this, it can be avoided that the energization stop process of the heater HT, which is performed when the number n has reached the upper limit value $n_{max}$, is performed before the inhalation time $T_L$ reaches the threshold value $TH_{PRD}$.

In step S201, the processor 14 resets each of the number of times k the current value $I_D$ of the resistor $R_D$ exceeds the first current value $TH_1$ and the number of times n the current value $I_D$ exceeds the second current value $TH_2$ to 0. Then, in step S202, the processor 14 detects the current value $I_D$ of the resistor $R_D$ based on the voltage generated in the resistor $R_D$. In step S203, the processor 14 determines whether the current value $I_D$ detected in step S202 is larger than the first current value $TH_1$. If the current value $I_D$ is larger than the first current value $TH_1$, the process advances to step S204, and the processor 14 counts up the number of times k (for example, adds "1" to the number of times k).

In step S205, the processor 14 determines whether the number of times k is equal to or larger than the upper limit value $k_{max}$. If the number of times k is smaller than the upper limit value $k_{max}$, the process returns to step S202. On the other hand, if the number of times k is equal to or larger than the upper limit value $k_{max}$, the processor 14 determines in step S206 that an overcurrent (temporary overcurrent) of the heater HT is detected, and stops the energization of the heater HT in step S207. The energization stop process of the heater HT in step S207 can be, for example, a process of turning off the switches Q1 and Q2 and/or a process of stopping the voltage converter 11 and the voltage conversion circuit 12 by setting the EN terminal of each of the voltage converter 11 and the voltage conversion circuit 12 to Low level. The process of turning off the switches Q1 and Q2 reduces the transient response of energization upon stopping the energization of the heater HT more than the process of stopping the voltage converter 11 and the voltage conversion circuit 12. Accordingly, it is possible to stop the temporary overcurrent more quickly. However, in a situation in which a temporary current is generated, there is a possibility that at least one of the switch Q1, the switch Q2, the voltage converter 11, and the voltage conversion circuit 12 has a problem. Therefore, by simultaneously performing the process of turning off the switches Q1 and Q2 and the process of stopping the voltage converter 11 and the voltage conversion circuit 12, it is possible to stop the temporary overcurrent more reliably. In this manner, step S205 corresponds to the constraint condition (second constraint condition) for restricting the energization of the heater HT. That is, if the constraint condition that the number of times k the current value $I_D$ of the resistor $R_D$ (the current value $I_{HTR}$ of the heater HT) exceeds the first current value $TH_1$ is equal to or larger than the upper limit value $k_{max}$ is met, the processor 14 stops the energization to the heather HT even if the atomization request is detected by the puff sensor 21 and/or the touch sensor 22.

If the current value $I_D$ is equal to smaller than the first current value $TH_1$ in step S203, the process advances to step S208. In step S208, the processor 14 determines whether the current value $I_D$ detected in step S202 is larger than the second current value $TH_2$. If the current value $I_D$ is larger than the second current value $TH_1$, the process advances to step S209, and the processor 14 counts up the number of times n (for example, adds "1" to the number of times n).

In step S210, the processor 14 determines whether the number of times n is equal to or larger than the upper

limit value $n_{max}$. If the number of times n is smaller than the upper limit value $n_{max}$, the process returns to step S202. On the other hand, if the number of times n is equal to or larger than the upper limit value $n_{max}$, the processor 14 determines in step S211 that an overcurrent (long-term overcurrent) of the heater HT is detected, and stops the energization of the heater HT in step S207. In this manner, step S210 corresponds to the constraint condition (second constraint condition) for restricting the energization of the heater HT. That is, if the constraint condition that the number of times n the current value $I_D$ of the resistor $R_D$ (the current value $I_{HTR}$ of the heater HT) exceeds the second current value $TH_2$ is equal to or larger than the upper limit value $n_{max}$ is met, the processor 14 stops the energization to the heather HT even if the atomization request is detected by the puff sensor 21 and/or the touch sensor 22.

Here, the processor 14 may perform the energization inhibition process of inhibiting the energization to the heater HT in step S207. The energization inhibition process can include, for example, a process of inhibiting the voltage converter 11 from outputting the heater driving voltage V1 and/or a process of inhibiting turn-on of the switch Q1 (that is, connecting the power supply line of the heater HT at the switch Q1). As in the case for a temporary overcurrent, the process of inhibiting turn-on of the switch Q1 can stop a long-term overcurrent more quickly than the process of inhibiting the voltage converter 11 from outputting the heater driving voltage V1. By simultaneously performing the process of inhibiting turn-on of the switch Q1 and the process of inhibiting the voltage converter 11 from outputting the heater driving voltage V1, it is possible to stop the long-term overcurrent more reliably. The energization inhibition process may be performed for a predetermined period (that is, the inhibition of the energization to the heater HT may be maintained for the predetermined period). Further, in step S207, in addition to stopping the energization of the heater HT, a notification indicating that an error (abnormality) has occurred in the controller 102 may be made. For example, this notification can be lighting or blinking of the LED 19 by the LED driving circuit 18.

In addition to the processor 14, the protection circuit 17 may perform the second detection-related process. If a plurality of elements can independently detect an overcurrent of the heater HT and independently stop the overcurrent, the possibility of stopping the overcurrent is increased even if some elements have problems. Particularly, since an overcurrent is sometimes generated due to problems of some elements, the countermeasure as described above is important. As an example, when an overcurrent is generated due to a problem of the processor 14, there is a possibility that the processor 14 having the problem cannot stop the overcurrent. If the protection circuit 17 performs the second detection-related process, such the possibility can be effectively reduced. Note that when the protection circuit 17 performs the second detection-related process, the current value of the resistor $R_P$ may be used instead of the current value $I_D$ of the resistor $R_D$. The first current value $TH_1$, the second current value $TH_1$, the upper limit value $k_{max}$, and the upper limit value $n_{max}$ may be the same or different from those in the second detection-related process performed by the processor 14. If an overcurrent of the heater HT is detected, the protection circuit 17 may turn off the switch circuit SP.

Each of the resistor $R_D$ and the resistor $R_P$ connected between the second electrical contact C2 and the power supply BAT is used by the processor 14 and the protection circuit 17, respectively, to detect the current flowing through the heater HT. Instead, the resistor $R_D$ and the resistor $R_P$ may be connected between the power supply BAT and the first electrical contact C1. In a mode in which the resistor $R_D$ and the resistor $R_P$ are connected between the second electrical contact C2 and the power supply BAT, the voltage Vb supplied from the plus terminal of the power supply BAT or the voltage obtained by stepping down the heater driving voltage V1 by the heater HT or the like is applied to the resistor $R_D$ and the resistor $R_P$. Thus, when the processor 14 uses an amplifier to detect the voltage applied to the resistor $R_D$ and the protection circuit 17 uses an amplifier to detect the voltage applied to the resistor $R_P$, it is possible to lower the common-mode input voltage of the amplifies. That is, it is unnecessary to use a high-breakdown voltage amplifier which is generally expensive and large in size, so that the size and cost of the controller 102 or the inhalation device 100 can be reduced.

The invention is not limited to the foregoing embodiments, and various variations/changes are possible within the scope of the invention which is defined in the appended claims.

**Claims**

1. A controller (102) for an inhalation device (100), comprising:

   a sensor (21, 22) configured to detect an atomization request of an aerosol source; and
   a processor (14) configured to perform energization control of a heater (HT), which is used to heat the aerosol source, in accordance with the atomization request detected by the sensor,
   **characterized in that**
   the processor

      monitors a current flowing through the heater,
      determines whether each of three constraint conditions for the energization control is met, and

restricts, in a case where at least one of the three constraint conditions is met, energization to the heater even if the atomization request is detected by the sensor,

one of the three constraint conditions is a current constraint condition regarding a current value flowing through the heater, and
the current constraint condition includes a first current constraint condition for restricting energization to the heater in response to a temporary overcurrent of the heater exceeding a first threshold ($TH_1$),
and a second current constraint condition for restricting energization to the heater in response to an overcurrent of the heater exceeding a second threshold ($TH_2$) during stopping energization to the heater, and
the first threshold is larger than the second threshold.

2. The controller according to claim 1, **characterized in that**
monitoring targets for determining whether to restrict energization to the heater are different among the three constraint conditions.

3. The controller according to claim 1 or 2, **characterized in that**

the processor monitors a request continuation period ($T_L$) during which the atomization request is continuously detected by the sensor, and
another of the three constraint conditions is a request constraint condition regarding the request continuation period.

4. The controller according to any one of claims 1 to 3, further comprising

a first resistor (RD) provided on a power supply line to the heater,
**characterized in that** the processor monitors the current of the heater based on a voltage of the first resistor.

5. The controller according to any one of claims 1 to 4, **characterized in that**

the current constraint condition includes a first current constraint condition that a current exceeding a first current value flows through the heater in a time not shorter than a first time ($k_{max}/f$),
and a second current constraint condition that a current exceeding a second current value flows through the heater in a time not shorter than a second time ($n_{max}/f$), and
the first time is set shorter than the second time, and the first current value is set larger than the second current value.

6. The controller according to any one of claims 1 to 5, **characterized in that**

the processor monitors a temperature of the heater, and
another of the three constraint conditions is a temperature constraint condition regarding the temperature of the heater.

7. The controller according to claim 6, further comprising

an amplifier (13) configured to amplify and output a voltage generated in the heater,
**characterized in that** the processor monitors the temperature of the heater based on an output voltage of the amplifier.

8. The controller according to any one of claims 1 to 7, further comprising

a power supply (BAT); and
a voltage converter (11) configured to convert an output voltage of the power supply into a heater driving voltage, and output the heater driving voltage to a power supply line to the heater,
**characterized in that** the processor restricts energization of the heater by inhibiting the voltage converter from outputting the heater driving voltage.

9. The controller according to any one of claims 1 to 8, further comprising

a switch (Q1) provided on a power supply line to the heater, and configured to connect/disconnect the power supply line,
**characterized in that** the processor controls energization to the heater by inhibiting connecting the power supply line by the switch.

10. The controller according to any one of claims 1 to 9, further comprising:

a second resistor (RP) provided on a power supply line to the heater; and
a protection circuit (17) configured to stop energization to the heater in accordance with a voltage of the second resistor,
**characterized in that** the protection circuit operates independently of control of the processor.

11. The controller according to claim 10, further comprising

a switch circuit (SP) configured to connect/disconnect the power supply line,
**characterized in that** the protection circuit stops energization to the heater by disconnecting the power supply line by the switch circuit in accordance with the voltage of the second resistor.

12. The controller according to any one of claims 1 to 11, **characterized by**

a request constraint condition that the request continuation period during which the atomization request is continuously detected by the sensor is not shorter than a first threshold value ($TH_{PRD}$), and wherein
the current constraint condition includes a constraint condition that an energization time of the heater is not shorter than a second threshold value ($n_{max}/f$), and
the second threshold value is set longer than the first threshold value.

13. The controller according to any one of claims 1 to 12, **characterized in that**

the first current constraint condition is a constraint condition that a current exceeding the first threshold ($TH_1$) flows through the heater for a first time period ($k_{max}/f$) or longer,
the second current constraint condition is a constraint condition that a current exceeding the second threshold ($TH_2$) flows through the heater for a second time period ($n_{max}/f$) or longer, and
the first time period is shorter than the second time period.

14. The controller according to claim 13, **characterized by**

a request constraint condition that a request continuation period during which the atomization request is continuously detected by the sensor is longer than a period threshold ($TH_{PRD}$), and wherein
the first time period ($k_{max}/f$) is shorter than the period threshold, and the second time period ($n_{max}/f$) is longer than the period threshold.

15. The controller according to any one of claims 1 to 14, **characterized in that** the first threshold ($TH_1$) is set to a value larger than a normal current value ($I_{Q1}, I_{Q2}$) that can flow through the heater during energization to the heater in accordance with the atomization request, and the second threshold ($TH_2$) is set to a value smaller than the normal current value.

**Patentansprüche**

1. Steuergerät (102) für eine Inhalationsvorrichtung (100), umfassend:

einen Sensor (21, 22), der so konfiguriert ist, dass er eine Zerstäubungsanforderung einer Aerosolquelle erfasst; und
einen Prozessor (14), der so konfiguriert ist, dass er eine Speisungssteuerung eines Heizelements (HT), das zum Erhitzen der Aerosolquelle verwendet wird, in Übereinstimmung mit der vom Sensor erfassten Zerstäubungsanforderung durchführt,
**dadurch gekennzeichnet, dass**
der Prozessor

einen Strom überwacht, der durch das Heizelement fließt,
bestimmt, ob jede von drei Randbedingungen für die Speisungssteuerung erfüllt ist, und
in einem Fall, in dem mindestens eine der drei Randbedingungen erfüllt ist, die Speisung des Heizelements einschränkt, auch wenn die Zerstäubungsanforderung vom Sensor erfasst wird,

eine der drei Randbedingungen eine Stromrandbedingung ist, die sich auf einen Stromwert bezieht, der durch das Heizelement fließt, und
die Stromrandbedingung eine erste Stromrandbedingung zum Einschränken der Speisung des Heizelements als Reaktion auf einen vorübergehenden Überstrom des Heizelements, der einen ersten Schwellenwert ($TH_1$) überschreitet, und eine zweite Stromrandbedingung zum Einschränken der Speisung des Heizelements als Reaktion auf einen Überstrom des Heizelements, der einen zweiten Schwellenwert ($TH_2$) während des Anhaltens der Speisung des Heizelements überschreitet, einschließt, und
der erste Schwellenwert größer ist als der zweite Schwellenwert.

2. Steuergerät nach Anspruch 1, **dadurch gekennzeichnet, dass**
sich Überwachungsziele zum Bestimmen, ob die Speisung zum Heizelement eingeschränkt werden soll, unter den drei Randbedingungen unterscheiden.

3. Steuergerät nach Anspruch 1 oder 2, **dadurch gekennzeichnet, dass**

der Prozessor eine Anforderungsfortsetzungsperiode ($T_L$) überwacht, während der die Zerstäubungsanforderung kontinuierlich von dem Sensor erfasst wird, und
eine weitere der drei Randbedingungen eine Anforderungsrandbedingung ist, die sich auf die Anforderungsfortsetzungsperiode bezieht.

4. Steuergerät nach einem der Ansprüche 1 bis 3, weiter umfassend

einen ersten Widerstand (RD), der in einer Stromversorgungsleitung zum Heizelement bereitgestellt ist,
**dadurch gekennzeichnet, dass** der Prozessor den Strom des Heizelements auf der Grundlage einer Spannung des ersten Widerstands überwacht.

5. Steuergerät nach einem der Ansprüche 1 bis 4, **dadurch gekennzeichnet, dass**

die Stromrandbedingung eine erste Stromrandbedingung einschließt, dass ein Strom, der einen ersten Stromwert überschreitet, in einer Zeit, die nicht kürzer als eine erste Zeit ($k_{max}/f$) ist, durch das Heizelement fließt, und eine zweite Stromrandbedingung, dass ein Strom, der einen zweiten Stromwert überschreitet, in einer Zeit, die nicht kürzer als eine zweite Zeit ($n_{max}/f$) ist, durch das Heizelement fließt, und
die erste Zeit kürzer als die zweite Zeit eingestellt ist und der erste Stromwert größer als der zweite Stromwert eingestellt ist.

6. Steuergerät nach einem der Ansprüche 1 bis 5, **dadurch gekennzeichnet, dass**

der Prozessor eine Temperatur des Heizelements überwacht, und
eine weitere der drei Randbedingungen eine Temperaturrandbedingung ist, die sich auf die Temperatur des Heizelements bezieht.

7. Steuergerät nach Anspruch 6, weiter umfassend

einen Verstärker (13), der so konfiguriert ist, dass er eine in dem Heizelement erzeugte Spannung verstärkt und ausgibt,
**dadurch gekennzeichnet, dass** der Prozessor die Temperatur des Heizelements auf der Grundlage einer Ausgangsspannung des Verstärkers überwacht.

8. Steuergerät nach einem der Ansprüche 1 bis 7, weiter umfassend

eine Stromversorgung (BAT); und
einen Spannungswandler (11), der so konfiguriert ist, dass er eine Ausgangsspannung der Stromversorgung in eine Heizungsansteuerspannung umwandelt und die Heizungsansteuerspannung an eine Stromversorgungsleitung zum Heizelement ausgibt,
**dadurch gekennzeichnet, dass** der Prozessor die Speisung des Heizelements einschränkt, indem er den Spannungswandler daran hindert, die Heizungsansteuerspannung auszugeben.

9. Steuergerät nach einem der Ansprüche 1 bis 8, weiter umfassend

einen Schalter (Q1), der an einer Stromversorgungsleitung zum Heizelement bereitgestellt und so konfiguriert ist, dass er die Stromversorgungsleitung verbindet/trennt,
**dadurch gekennzeichnet, dass** der Prozessor die Speisung des Heizelements steuert, indem er den Anschluss der Stromversorgungsleitung durch den Schalter verhindert.

10. Steuergerät nach einem der Ansprüche 1 bis 9, weiter umfassend:

einen zweiten Widerstand (RP), der in einer Stromversorgungsleitung zum Heizelement bereitgestellt ist; und
eine Schutzschaltung (17), die so konfiguriert ist, dass sie die Speisung des Heizelements in Übereinstimmung mit einer Spannung des zweiten Widerstands unterbricht,
**dadurch gekennzeichnet, dass** die Schutzschaltung unabhängig von der Steuerung durch den Prozessor arbeitet.

11. Steuergerät nach Anspruch 10, weiter umfassend

einen Schaltkreis (SP), der so konfiguriert ist, dass er die Stromversorgungsleitung verbindet/trennt,
**dadurch gekennzeichnet, dass** die Schutzschaltung die Speisung des Heizelements unterbricht, indem sie die Stromversorgungsleitung durch den Schaltkreis in Übereinstimmung mit der Spannung des zweiten Widerstands trennt.

12. Steuergerät nach einem der Ansprüche 1 bis 11, **gekennzeichnet durch** eine Anforderungsrandbedingung, dass die Anforderungsfortsetzungsperiode, während der die Zerstäubungsanforderung kontinuierlich von dem Sensor erfasst wird, nicht kürzer als ein erster Schwellenwert ($TH_{PRD}$) ist, und wobei

die Stromrandbedingung eine Randbedingung einschließt, dass eine Speisungszeit des Heizelements nicht kürzer als ein zweiter Schwellenwert ($n_{max}/f$) ist, und
der zweite Schwellenwert länger als der erste Schwellenwert eingestellt ist.

13. Steuergerät nach einem der Ansprüche 1 bis 12, **dadurch gekennzeichnet, dass**

die erste Stromrandbedingung eine Randbedingung ist, dass ein Strom, der den ersten Schwellenwert ($TH_1$) überschreitet, für eine erste Zeitspanne ($k_{max}/f$) oder länger durch das Heizelement fließt,
die zweite Stromrandbedingung eine Randbedingung ist, dass ein Strom, der den zweiten Schwellenwert ($TH_2$) überschreitet, für eine zweite Zeitspanne ($n_{max}/f$) oder länger durch das Heizelement fließt, und
die erste Zeitspanne kürzer ist als die zweite Zeitspanne.

14. Steuergerät nach Anspruch 13, **gekennzeichnet durch** eine Anforderungsrandbedingung, dass eine Anforderungsfortsetzungsperiode, während der die Zerstäubungsanforderung kontinuierlich durch den Sensor erfasst wird, länger ist als ein Periodenschwellenwert ($TH_{PRD}$),
und wobei die erste Zeitspanne ($k_{max}/f$) kürzer ist als der Periodenschwellenwert und die zweite Zeitspanne ($n_{max}/f$) länger ist als der Periodenschwellenwert.

15. Steuergerät nach einem der Ansprüche 1 bis 14, **dadurch gekennzeichnet, dass** der erste Schwellenwert ($TH_1$) auf einen Wert eingestellt ist, der größer ist als ein normaler Stromwert ($I_{Q1}, I_{Q2}$), der durch das Heizelement während der Speisung des Heizelements in Übereinstimmung mit der Zerstäubungsanforderung fließen kann, und der zweite Schwellenwert ($TH_2$) auf einen Wert eingestellt ist, der kleiner ist als der normale Stromwert.

**Revendications**

1. Organe de commande (102) pour un dispositif d'inhalation (100), comprenant :

un capteur (21, 22) configuré pour détecter une demande d'atomisation d'une source d'aérosol ; et
un processeur (14) configuré pour mettre en oeuvre une commande de fourniture d'énergie d'un appareil de chauffage (HT), qui est utilisé pour chauffer la source d'aérosol, en fonction de la demande d'atomisation détectée par le capteur,
**caractérisé en ce que**
le processeur

surveille un courant circulant à travers l'appareil de chauffage,
détermine si oui ou non chacune des trois conditions de contrainte pour la commande de fourniture d'énergie est remplie, et
restreint, dans un cas dans lequel au moins une des trois conditions de contrainte est remplie, la fourniture d'énergie à l'appareil de chauffage même si la demande d'atomisation est détectée par le capteur,

l'une des trois conditions de contrainte est une condition de contrainte de courant concernant une valeur de courant circulant à travers l'appareil de chauffage, et
la condition de contrainte de courant inclut une première condition de contrainte de courant destinée à restreindre la fourniture d'énergie à l'appareil de chauffage en réponse au fait qu'une surintensité temporaire de l'appareil de chauffage dépasse un premier seuil ($TH_1$), et une seconde condition de contrainte de courant destinée à restreindre la fourniture d'énergie à l'appareil de chauffage en réponse au fait qu'une surintensité de l'appareil de chauffage dépasse un second seuil ($TH_2$) pendant l'arrêt de la fourniture d'énergie à l'appareil de chauffage, et
le premier seuil est supérieur au second seuil.

2. Organe de commande selon la revendication 1, **caractérisé en ce que**
des cibles de surveillance destinées à déterminer s'il convient ou non de restreindre la fourniture d'énergie à l'appareil de chauffage sont différentes parmi les trois conditions de contrainte.

3. Organe de commande selon la revendication 1 ou la revendication 2, **caractérisé en ce que**

le processeur surveille une période de continuation de demande ($T_L$) pendant laquelle la demande d'atomisation est détectée en continu par le capteur, et
une autre des trois conditions de contrainte est une condition de contrainte de demande concernant la période de continuation de demande.

4. Organe de commande selon l'une quelconque des revendications 1 à 3, comprenant en outre

une première résistance (RD) fournie sur une ligne d'alimentation électrique de l'appareil de chauffage,
**caractérisé en ce que** le processeur surveille le courant de l'appareil de chauffage sur la base d'une tension de la première résistance.

5. Organe de commande selon l'une quelconque des revendications 1 à 4, **caractérisé en ce que**

la condition de contrainte de courant inclut une première condition de contrainte de courant selon laquelle un courant dépassant une première valeur de courant circule à travers l'appareil de chauffage sur une durée qui n'est pas plus courte qu'une première durée ($k_{max}/f$),
et une seconde condition de contrainte selon laquelle un courant dépassant une seconde valeur de courant circule à travers l'appareil de chauffage sur une durée qui n'est pas plus courte qu'une seconde durée ($n_{max}/f$), et
la première durée est définie de manière à être plus courte que la seconde durée, et la première valeur de courant est définie de manière à être supérieure à la seconde valeur de courant.

6. Organe de commande selon l'une quelconque des revendications 1 à 5, **caractérisé en ce que**

le processeur surveille une température de l'appareil de chauffage, et
une autre des trois conditions de contrainte est une condition de contrainte de température concernant la température de l'appareil de chauffage.

7. Organe de commande selon la revendication 6, comprenant en outre

un amplificateur (13) configuré pour amplifier et émettre en sortie une tension générée dans l'appareil de chauffage,
**caractérisé en ce que** le processeur surveille la température de l'appareil de chauffage sur la base d'une tension de sortie de l'amplificateur.

8. Organe de commande selon l'une quelconque des revendications 1 à 7, comprenant en outre

une alimentation électrique (BAT) ; et
un convertisseur (11) de tension configuré pour convertir une tension de sortie de l'alimentation électrique en une tension d'entraînement d'appareil de chauffage, et émettre en sortie la tension d'entraînement d'appareil de chauffage vers une ligne d'alimentation électrique de l'appareil de chauffage,
**caractérisé en ce que** le processeur restreint la fourniture d'énergie à l'appareil de chauffage en inhibant la sortie par le convertisseur de tension de la tension d'entraînement d'appareil de chauffage.

9. Organe de commande selon l'une quelconque des revendications 1 à 8, comprenant en outre

un commutateur (Q1) fourni sur une ligne d'alimentation électrique de l'appareil de chauffage, et configuré pour connecter/déconnecter la ligne d'alimentation électrique,
**caractérisé en ce que** le processeur commande la fourniture d'énergie à l'appareil de chauffage en inhibant la connexion de la ligne d'alimentation électrique par le commutateur.

10. Organe de commande selon l'une quelconque des revendications 1 à 9, comprenant en outre :

une seconde résistance (RP) fournie sur une ligne d'alimentation électrique de l'appareil de chauffage ; et
un circuit de protection (17) configuré pour mettre fin à la fourniture d'énergie à l'appareil de chauffage en fonction d'une tension de la seconde résistance,
**caractérisé en ce que** le circuit de protection fonctionne indépendamment de la commande du processeur.

11. Organe de commande selon la revendication 10, comprenant en outre

un circuit de commutation (SP) configuré pour connecter/déconnecter la ligne d'alimentation électrique,
**caractérisé en ce que** le circuit de protection met fin à la fourniture d'énergie à l'appareil de chauffage en déconnectant la ligne d'alimentation électrique par le circuit de commutation en fonction de la tension de la seconde résistance.

12. Organe de commande selon l'une quelconque des revendications 1 à 11, **caractérisé par** une condition de contrainte de demande selon laquelle la période de continuation de demande pendant laquelle la demande d'atomisation est détectée en continu par le capteur n'est pas inférieure à une première valeur seuil ($TH_{PRD}$), et dans lequel

la condition de contrainte de courant inclut une condition de contrainte selon laquelle un temps de fourniture d'énergie de l'appareil de chauffage n'est pas inférieur à une seconde valeur seuil ($n_{max}/f$), et
la seconde valeur seuil est définie de manière à être supérieure à la première valeur seuil.

13. Organe de commande selon l'une quelconque des revendications 1 à 12, **caractérisé en ce que**

la première condition de contrainte de courant est une condition de contrainte selon laquelle un courant dépassant le premier seuil ($TH_1$) circule à travers l'appareil de chauffage dans une première période ($k_{max}/f$) ou une période plus longue que celle-ci,
la seconde condition de contrainte de courant est une condition de contrainte selon laquelle un courant dépassant le second seuil ($TH_2$) circule à travers l'appareil de chauffage dans une seconde période ($n_{max}/f$) ou une période plus longue que celle-ci, et
la première période est plus courte que la seconde période.

14. Organe de commande selon la revendication 13, **caractérisé par** une condition de contrainte de demande selon laquelle une période de continuation de demande pendant laquelle la demande d'atomisation est détectée en continu par le capteur est supérieure à un seuil de période ($TH_{PRD}$),
et dans lequel la première période ($k_{max}/f$) est inférieure au seuil de période, et la seconde période ($n_{max}/f$) est supérieure au seuil de période.

**15.** Organe de commande selon l'une quelconque des revendications 1 à 14, **caractérisé en ce que** le premier seuil ($TH_1$) est défini à une valeur supérieure à une valeur de courant normale ($I_{Q1}I_{Q2}$) qui peut circuler à travers l'appareil de chauffage pendant la fourniture d'énergie à l'appareil de chauffage en fonction de la demande d'atomisation, et le second seuil ($TH_2$) est défini à une valeur inférieure à la valeur de courant normale.

# F I G. 1

**FIG. 2**

# F I G. 3

START

S11 — IS THERE ATOMIZATION REQUEST? — NO

YES

S12 — Vb > Vend? — NO

YES

S29 — LIGHT LED IN RED

END

S13 — LIGHT LED IN BLUE

S14 — START SUPPLYING POWER

S15 — INHALATION TIME $T_L$ = 0

S16 — $T_L = T_L + \Delta t$

S17 — HAS ATOMIZATION REQUEST FINISHED? — NO

S18 — $T_L \geq$ UPPER LIMIT TIME? — NO

YES

YES

S19 — STOP SUPPLYING POWER

S20 — TURN OFF LED

S21 — ACCUMULATED TIME $T_A = T_A + T_L$

S22 — $T_A >$ INHALATION ENABLE TIME? — NO

YES

S23 — IS THERE ATOMIZATION REQUEST? — NO

YES

S24 — CONTINUE FOR PREDETERMINED TIME? — NO

YES

S25 — INHIBIT SUPPLYING POWER

S26 — BLINK LED IN BLUE

S27 — EXCHANGE COMPLETE? — NO

YES

S28 — CANCEL INHIBITION OF POWER SUPPLY

EP 3 964 086 B1

F I G.  4

START

START DETECTION OF
ATOMIZATION REQUEST? — S101

NO

YES

ACTIVATE FIRST REGULATOR AND
SECOND REGULATOR — S102

TURN ON SWITCH Q1
(START HEATING) — S103

TURN ON SWITCH Q2 — S104

TURN OFF SWITCH Q1
(STOP HEATING) — S105

MEASURE HEATER TEMPERATURE — S106

TURN OFF SWITCH Q2 — S107

S108
HEATER TEMPERATURE <
THRESHOLD $TH_{TMP}$?

NO

YES

S110
END DETECTION OF
ATOMIZATION REQUEST?

YES

NO

S111
INHALATION TIME ≥
THRESHOLD $TH_{PRD}$?

NO

YES

S109
ERROR PROCESS

STOP FIRST REGULATOR AND
SECOND REGULATOR — S112

END

# F I G. 5

```
                          ┌─────────────┐
                          │    START    │
                          └──────┬──────┘
                                 ▼
                   ┌──────────────────────┐ ⟋ S201
                   │        k, n = 0       │
                   └───────────┬──────────┘
                               ▼
              ┌───────────────────────────┐ ⟋ S202
              │  DETECT CURRENT VALUE $I_D$ │
              └─────────────┬─────────────┘
                            ▼         S203
                      ◇───────────◇
                     ╱  $I_D$ > $TH_1$? ╲───── NO
                      ◇───────────◇
                            │ YES                    ◇───────────◇ S208
                            ▼                       ╱ $I_D$ > $TH_2$? ╲──── NO
                   ┌────────────┐ S204               ◇───────────◇
                   │    k++     │                         │ YES
                   └─────┬──────┘                         ▼
                         ▼        S205          ┌────────────┐ S209
               ◇──────────────◇                │    n++     │
          NO ─╱  k ≥ $k_{max}$?  ╲              └─────┬──────┘
               ◇──────────────◇                       ▼       S210
                     │ YES                   ◇──────────────◇
                     ▼                      ╱  n ≥ $n_{max}$?  ╲── NO
         ┌────────────────────┐ S206         ◇──────────────◇
         │  DETECT OVERCURRENT │                   │ YES
         │    (ABNORMALITY)    │                   ▼
         └──────────┬─────────┘        ┌────────────────────┐ S211
                    ▼                  │  DETECT OVERCURRENT │
         ┌────────────────────┐ S207   │    (ABNORMALITY)    │
         │   STOP ENERGIZATION │        └────────────────────┘
         │     OF HEATER       │
         └──────────┬─────────┘
                    ▼
              ┌──────────┐
              │   END    │
              └──────────┘
```

# F I G. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019180909 A1

- JP 2020058337 A